# EUROPÄISCHE PATENTSCHRIFT

(11) **EP 3 492 143 B1**
(45) Veröffentlichungstag und Bekanntmachung des Hinweises auf die Patenterteilung: **10.03.2021**
(21) Anmeldenummer: 17204327.5
(22) Anmeldetag: 29.11.2017
(51) Int. Cl.: A61Q 11/00, A61K 8/73, A61K 8/04

(54) **NIEDRIGVISKOSE ZAHNPFLEGEZUSAMMENSETZUNG**
LOW VISCOSITY DENTAL CARE COMPOSITION
COMPOSITION DE SOINS DENTAIRES À BASSE VISCOSITÉ

(43) Veröffentlichungstag der Anmeldung: 05.06.2019
(60) Teilanmeldung: 20193996.4
(73) Patentinhaber: BLBR GmbH, 82031 Grünwald (DE)
(72) Erfinder: Sörgel, Norbert, 82031 Gründwald (DE); Keiner, Michael, 35619 Braunfels (DE)
(74) Vertreter: Becker & Kurig Partnerschaft Patentanwälte PartmbB

(56) Entgegenhaltungen:
- WO-A1-2012/015420
- DE-A1-102015 109 891
- JP-A- S5 585 513
- JP-A- 2016 175 869
- US-A1- 2004 126 331
- US-A1- 2012 180 806

## Beschreibung

### GEBIET DER ERFINDUNG

Die vorliegende Erfindung betrifft eine niedrigviskose Mund-, Zahnpflege- und Zahnreinigungszusammensetzung, welche zur Anwendung bei der Zahnreinigung und -pflege mittels einer Zahnputzvorrichtung, insbesondere einer elektrischen Schall- bzw. Ultraschallzahnbürste, für die gleichzeitige Reinigung mehrerer, vorzugsweise aller Zähne eines Benutzers, geeignet ist. Durch ihre niedrige Viskosität kann die Zusammensetzung gleichmäßig, schnell und effizient auf den Mundeinsatz der Zahnputzvorrichtung, z.B. auf Reinigungsprothesen oder sonstige Vorrichtungen, die zum gleichzeitigen Reinigen mehrerer oder aller Zähne dienen, aufgetragen und verteilt werden. Die Handhabung wird dadurch wesentlich vereinfacht.

### STAND DER TECHNIK

Generell liegen Mund- und Zahnpflegemittel in Pasten- oder Cremeform vor. Sie haben eine Viskosität von 60.000 bis 80.000 mPas (Millipascalsekunden; gemessen bei 20 °C) und werden aus Tuben oder Standbehältem durch manuelles Zusammendrücken des Behälters auf den Bürstenkopf einer Zahnbürste abgegeben. Die übliche Anwendungsmenge liegt bei 1 bis 2 ml pro Benutzung. In selteneren Fällen liegt das Mund- und Zahnpflegemittel in Gelform mit einer Viskosität von 10.000 bis 70.000 mPas (bei 20°C) vor. Auch hier wird eine Menge von 1 bis 2 ml durch Druck auf den Spenderbehälter auf den Bürstenkopf einer Zahnbürste appliziert. Eine Zahncreme mit relativ niedriger Viskosität ist in EP 2813214 A1 offenbart.

US 2004/0126331 A1 beschreibt eine niedrigviskose Zusammensetzungen zur Zahnreinigung, die für die kleinen Bürstenköpfe einer elektrischen Zahnbürste geeignet ist.

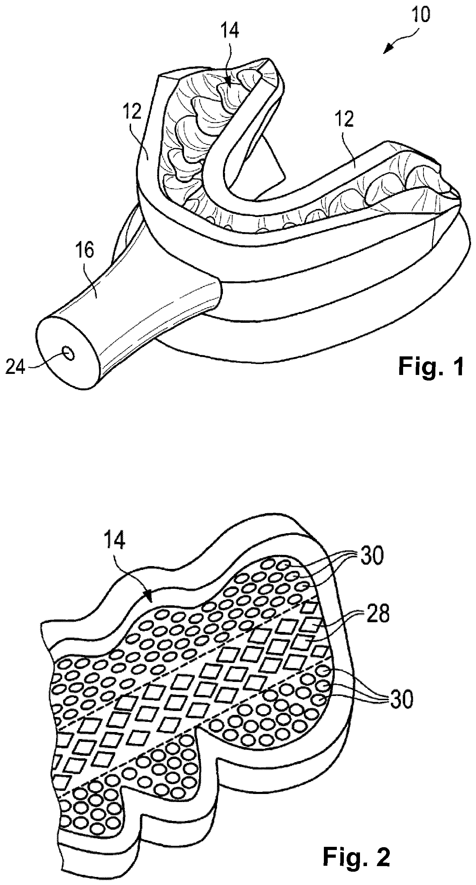

Die vorliegende Erfindung ist jedoch nicht auf die Anwendung der Mund- und Zahnpflege- und -reinigungszusammensetzung mit einer herkömmlichen Handzahnbürste oder einer üblichen elektrischen Zahnbürste gerichtet. Vielmehr gibt es neuere Entwicklungen auf dem Gebiet der Zahnreinigung mittels eines Mundeinsatzes oder mehrerer Mundeinsätze, die zum gleichzeitigen Reinigen mehrerer oder aller Zähne dienen. Diese Mundeinsätze (siehe z.B. Fig. 1) sind zumindest grob, vorzugsweise individuell, an die Gebissform eines Benutzers angepasst und in der Lage, durch einen Motor zu Vibrationen angeregt, mehrere oder gar alle Zähne gleichzeitig zu reinigen. Entsprechende Zahnreinigungssysteme sind etwa in DE 10 2015 109891 A1, WO 2009/137671 A1 oder WO 2015/003681 A1 offenbart. Im Sinne der vorliegenden Erfindung bedeutet die gleichzeitige Reinigung mehrerer Zähne, dass der Mundeinsatz so ausgebildet ist, dass vorzugsweise gleichzeitig mindestens 3, noch mehr bevorzugt mindestens 5, noch mehr bevorzugt mindestens 7 Zähne gereinigt werden können. Ganz besonders bevorzugt ist der bzw. sind die Mundeinsätze so ausgebildet, dass gleichzeitig alle Zähne des Oberkiefers und/oder des Unterkiefers gereinigt werden können. Gleichzeitig bedeutet, dass der oder die Mundeinsätze zu einem Zeitpunkt T1 mit allen zu reinigenden Zähnen in Kontakt stehen und diese reinigen. Im Unterschied dazu tritt der Bürstenkopf einer herkömmlichen manuellen oder elektrischen Zahnbüste zeitlich nacheinander mit den zu reinigenden Zähnen in Kontakt.

Für die Benutzung in solchen Mundeinsatzelementen sind die vorhandenen Darreichungsformen von Zahnreinigungs- und -pflegeprodukten, wie beispielsweise Zahnpasten, Zahncremes oder Zahngele, jedoch ungeeignet. Die Reinigungs- und Pflegemittel gemäß dem Stand der Technik müssten mit einer Bürste oder einem Spatel auf den Reinigungsflächen der Mundeinsatzelemente verteilt werden, wobei die hohe Viskosität herkömmlicher Zahnpasten, Zahncremes oder -gele eine gleichmäßige Verteilung nahezu unmöglich macht. Gleichzeitig würde die Menge der zu verwendenden Creme, Paste oder des Gels auf ca. 20 bis 25 ml ansteigen, was zu einer übermäßigen Verschwendung von Ressourcen und einer Überdosierung führen würde.

### KURZE BESCHREIBUNG DER ERFINDUNG

Die vorliegende Erfindung betrifft gemäß einem ersten Aspekt eine niedrigviskose Mund-, Zahnpflege- und Zahnreinigungszusammensetzung mit einer Viskosität im Bereich von 50 - 10.000 m Pa s bei 20°C (1013,25 mbar, SVM™ 3001 Stabinger Viskosimeter™ von Anton Paar nach ASTM D7042).

Die niedrigviskose Mund-, Zahnpflege- und Zahnreinigungszusammensetzung, im Folgenden auch als Zusammensetzung oder Zahnpflegezusammensetzung bezeichnet, umfasst
- 20 bis 40 Gew.-% wenigstens eines Putzkörpers,
- 30 bis 45 Gew.-% wenigstens eines Feuchthaltemittels wobei das Feuchthaltemittel ausgewählt ist aus der Gruppe, bestehend aus Glycerin, Sorbitol, Xylit und beliebigen Mischungen davon,
- 0,8 bis 3,5 Gew.-% wenigstens eines Viskositätsreglers und Schaumbildners,
- 0,5 bis 1,8 Gew.-% wenigstens eines Schaumstabilisators,
- 3,5 bis 8 Gew.-% wenigstens eines Wirkstoffs, insbesondere wenigstens eines Fluorid-Wirkstoffs, wenigstens eines Mittels zur Zahnfleischpflege und/oder wenigstens eines desinfizierenden Bestandteils, beispielsweise eines Bakterizides,
- 0 bis 1,0 Gew.-%, vorzugsweise 0,3 bis 1,0 Gew.-%, wenigstens eines Farbpigments,
- 0 bis 1,5 Gew.-%, vorzugsweise 0,5 bis 1,5 Gew%, wenigstens eines Aromastoffs, und
- Lösungsmittel ad 100 Gew.-%.

Die erfindungsgemäße Zusammensetzung ist besonders zur Anwendung bei der Zahnreinigung und -pflege mittels einer Zahnputzvorrichtung für die gleichzeitige Reinigung mehrerer, vorzugsweise aller Zähne eines Benutzers, geeignet, wobei die Zusammensetzung durch Aufbringen der Zusammensetzung auf einen Mundeinsatz oder mehrere Mundeinsätze der Zahnputzvorrichtung dargereicht werden kann.

Die niedrige Viskosität der Zusammensetzung ermöglicht eine gleichmäßige und sparsame Verteilung der Zusammensetzung auf allen Reinigungsflächen des Mundeinsatzes oder der Mundeinsätze.

In einer bevorzugten erfindungsgemäßen Ausführungsform ist die Zusammensetzung als Schaum, vorzugsweise als Schaumspray, oder als Aerosol formuliert. Ein Schaum im Sinne der vorliegenden Erfindung ist eine feine oder kolloide Verteilung eines Gases in einer Flüssigkeit, im vorliegenden Fall also der hierin beschriebenen niedrigviskosen Zahnpflegezusammensetzung. Ein Schaumspray ist eine Emissionsform, in der zunächst ein Schaum durch eine Schaumdüse gebildet wird, der dann noch in der Sprayvorrichtung durch einen Gasstrom zerrissen wird und somit kleinere Schaumtropfen auf die Zielfläche emittiert werden und dort gleichmäßig verteilt werden können. Eine Darreichungsform der Zusammensetzung als Schaum, vorzugsweise als Schaumspray, ist erfindungsgemäß besonders bevorzugt.

In einer erfindungsgemäßen Ausführungsform wird die Zusammensetzung in einem Behälter bereitgestellt, der ein Fördersystem zum Befördern der Zusammensetzung nach außen, ein Ventil und eine Schaumdüse umfasst.

Ähnliche Vorrichtungen sind zwar bereits bekannt, jedoch nicht für eine Bereitstellung oder Anwendung einer Zusammensetzung gemäß der vorliegenden Offenbarung. Bekannte Bereitstellungen und Anwendungen sind beispielsweise Haarsprays, Deo-Sprays, Rasierschäume, Rasiergele, Bodymousses, Enthaarungsmousses, Haartönungsmousses, Schaumfestiger, Desinfektionssprays, Fußsprays, Sonnenschutzschäume, Anti-Flecken-Schaumsprays, Kunstschneesprays und Kühlsprays.

In einer erfindungsgemäßen Ausführungsform befördert das Fördersystem die Zusammensetzung mittels eines Treibgases, Druckluft oder über ein Pumpsystem aus dem Behälter. Die Austrittsöffnung des Behälters, beispielsweise die Schaumdüse, ist vorzugsweise derart ausgestaltet, dass eine gleichmäßige, zielgenaue und sparsame Verteilung der erfindungsgemäßen Zusammensetzung auf dem oder den Mundeinsätzen gewährleistet wird.

Eine niedrigviskose Mund-, Zahnpflege- und Zahnreinigungszusammensetzung, die die üblichen pharmazeutisch/kosmetisch akzeptablen Inhalts- und Zusatz- oder Hilfsstoffe enthält, mit einer Viskosität von 50 - 10.000 m Pa s (bei 20°C, 1013,25 mbar, SVM™ 3001 Stabinger Viskosimeter™ von Anton Paar nach ASTM D7042) kann zur Verwendung in einem Verfahren zur Zahnreinigung und -pflege mittels einer Vibrationszahnputzvorrichtung für die gleichzeitige Reinigung mehrerer, vorzugsweise aller Zähne eines Benutzers verwendet werden, wobei die Zusammensetzung dargereicht wird durch Aufbringen der Zusammensetzung auf einen Mundeinsatz oder mehrere Mundeinsätze der Zahnputzvorrichtung.

Die Inhalts- und Zusatz- oder Hilfsstoffe können ausgewählt sein aus der Gruppe, bestehend aus Viskositätsreglern, Feuchthaltemitteln, Putzkörper, Bindemitteln, Lösungsvermittlern, Vernetzungsmitteln, Tensiden, Aromastoffen, Stabilisatoren, Fluorid-Wirkstoffen, Zahnfleischpflegemitteln, Geschmacksstoffen und Farbpigmente, und aus beliebigen Kombinationen davon.

Hierfür ist die hierin beschriebene erfindungsgemäße Zusammensetzung ganz besonders geeignet. Sie stellt nicht nur eine ausreichende Wirkstoffmenge zur Verfügung, sondern kann darüber hinaus besonders gleichmäßig und einfach auf dem oder den Mundeinsätzen verteilt werden. Auch wird eine Überdosierung, wie sie bei herkömmlichen Zahncremes, -pasten oder -gelen auftreten würde, vermieden.

Die Zusammensetzung kann bevorzugt als Schaum, vorzugsweise als Schaumspray, oder als Aerosol auf den Mundeinsatz oder die Mundeinsätze aufgebracht werden.

Durch diese Applikation der Zusammensetzung auf den Mundeinsatz oder die Mundeinsätze wird die gleichmäßige und sparsame Verteilung der Zusammensetzung für den Benutzer weiter erleichtert und es kann eine gute Reinigung und Pflege der Zähne und des Zahnfleisches sichergestellt werden. Zudem weist ein Schaum (direkt oder als Schaumspray) eine gewisse Lebensdauer auf bevor er sich in seine niedrigviskose Grundzusammensetzung auflöst. In dieser Zeit kann der Mundeinsatz bzw. können die Mundeinsätze in den Mund des Benutzers eingeführt werden. Der auf den Mundeinsatz aufgetragene Schaum wird dann durch den Raum zwischen den Zähnen und Mundeinsatz zerdrückt und gleichmäßig verteilt. Somit kann der Schaum sehr sparsam auf den Mundeinsatz oder die Mundeinsätze aufgetragen werden.

Ein zur Zahnreinigung und -pflege verwendbarer Mundeinsatz kann zur Reinigung aller Zähne zwei U-förmige Wannen zur Aufnahme der Zähne aufweisen, wobei die beiden Wannen zum Gebiss des Benutzers passen und in entgegengesetztem Richtungen geöffnet sind. Eine niedrigviskose Zusammensetzung kann daher beim Einsetzen des Mundeinsatzes aus der unteren Wanne herausfließen oder sich am Außenrand der unteren Wanne sammeln, was das Reinigungsergebnis, insbesondere an den Kauflächen der Zähne des Unterkiefers, negativ beeinträchtigen kann. Ein Schaum verbleibt hingegen durch seine inhärente Festigkeit längere Zeit in der Wanne, auch wenn diese mit der Öffnung nach unten gedreht wird.

Das Aufbringen der Zusammensetzung erfolgt vorzugsweise mit Hilfe eines der hierin beschriebenen Behälter durch den die erfindungsgemäße Zusammensetzung in einen Schaum, vorzugsweise ein Schaumspray, oder ein Aerosol überführt werden kann.

Die Zahnreinigung und -pflege kann weiterhin das Einführen des Mundeinsatzes oder der Mundeinsätze mit der Zusammensetzung, vorzugsweise in der Form eines Schaums, Schaumtropfen oder Aerosoltropfen, in den Mund eines Benutzers und das gleichzeitige Reinigen und Pflegen mehrerer, bevorzugt aller Zähne des Benutzers durch Vibration des Mundeinsatzes umfassen. Die exakten Ausführungen der Mundreinigung über unterschiedliche Reinigungsstrukturen an den Innenflächen des Mundeinsatzes, Vibrationsfrequenzen, Reinigungsprogramme und Anwendungsdauer sind in der vorliegenden Offenbarung nicht beschränkt.

Der Mundeinsatz kann bzw. die Mundeinsätze können im Mund des Patienten durch einen Motor zu Schwingungen/ Vibrationen mit einer Frequenz im Bereich von 20 bis 700 Hz, bevorzugt 25 bis 600 Hz angeregt werden.

Der Mundeinsatz kann eine Form aufweisen, die die Zähne eines Benutzers während der Benutzung umgibt, so dass während des Reinigungsvorgangs Kauflächen und Innen- und Außenflanken der Zähne jeweils einer Fläche des Mundeinsatzes gegenüberliegen, und wobei eine Vielzahl von Reinigungsstrukturen auf den Zähnen zugewandten Seiten des Mundeinsatzes vorgesehen sind.

Der Mundeinsatz kann individuell an die Gebissform des Benutzers angepasst sein. Diese Anpassung kann durch herkömmliche negativ-positiv-Abdrücke, Abscannen und anschließendes 3D-Drucken des Mundeinsatzes, oder andere geeignete Verfahren erfolgen. Bevorzugte Materialien für den Mundeinsatz sind biokompatible Kunststoffe, z.B. biokompatibles Polyamid.

Der Mundeinsatz kann einteilig oder, jeweils für eine Gebissober- und Unterseite, zweiteilig ausgebildet sein.

Eine niedrigviskose Mund-, Zahnpflege- und Zahnreinigungszusammensetzung mit einer Viskosität im Bereich von 50 - 10.000 m Pa s bei 20°C kann in einem Verfahren zum Vorbeugen und/oder Behandeln von Erkrankungen der Zähne oder Zahnhälse, insbesondere von Karies, Parodontose, Plaque, sowie von Erkrankungen des Zahnfleisches mittels einer Zahnputzvorrichtung für die gleichzeitige Reinigung mehrerer, vorzugsweise aller Zähne eines Benutzers verwendet werden, wobei die Zusammensetzung dargereicht wird durch Aufbringen der Zusammensetzung auf einen Mundeinsatz oder mehrere Mundeinsätze der Zahnputzvorrichtung.

Die Offenbarung ist ebenfalls auf ein Verfahren bzw. das Verwendungsverfahren einer niedrigviskosen Mund-, Zahnpflege- und Zahnreinigungszusammensetzung, die die üblichen pharmazeutisch/kosmetisch akzeptablen Inhalts- und Zusatz- oder Hilfsstoffe enthält, und eine Viskosität im Bereich von 50 - 10.000 m Pa s (bei 20°C, 1013,25 mbar, SVM™ 3001 Stabinger Viskosimeter™ von Anton Paar nach ASTM D7042), aufweist, gerichtet. Offenbart wird also auch ein Verfahren zum Reinigen und Pflegen der Zähne und/oder des Zahnfleisches eines Benutzers, insbesondere zum Vorbeugen und/oder Behandeln von Erkrankungen der Zähne, der Zahnhälse und/oder des Zahnfleisches. Das Verfahren gemäß der vorliegenden Offenbarung umfasst entsprechend das Bereitstellen einer niedrigviskosen Mund-, Zahnpflege- und Zahnreinigungszusammensetzung mit einer Viskosität von 50 - 10.000 m Pa s bei 20°C (1013,25 mbar, SVM™ 3001 Stabinger Viskosimeter™ von Anton Paar nach ASTM D7042), das Auftragen der niedrigviskosen Zusammensetzung auf einen Mundeinsatz oder mehrere Mundeinsätze, der bzw. die für die Reinigung mehrerer, bevorzugt aller Zähne eines Benutzers geeignet ist/sind, das Einführen des Mundeinsatzes bzw. der Mundeinsätze mit der Zusammensetzung in den Mund des Benutzers und das Reinigen mehrerer, bevorzugt aller Zähne des Benutzers mit dem/den Mundeinsatz/ Mundeinsätzen und der Zusammensetzung. Das Reinigen erfolgt vorzugsweise dadurch, dass der oder die Mundeinsätze mittels einer Antriebsvorrichtung zur Vibration angeregt werden. Die hierin beschriebene Zusammensetzung kann ebenso im Bereich der Mund- und Zahnhygiene verwendet werden. Bevorzugt wird die Zusammensetzung mittels einer Zahnputzvorrichtung für die gleichzeitige Reinigung mehrerer, vorzugsweise aller Zähne eines Benutzers appliziert. Die Zusammensetzung kann dargereicht werden durch Aufbringen der Zusammensetzung auf einen Mundeinsatz oder mehrere Mundeinsätze der Zahnputzvorrichtung.

Für das Verfahren und die Verwendung ist die hierin beschriebene erfindungsgemäße Zusammensetzung aus den vorstehend genannten Gründen ganz besonders geeignet. Sie wird vorzugsweise mit Hilfe eines der hierin beschriebenen Behälter auf den oder die Mundeinsätze appliziert.

### KURZE BESCHREIBUNG DER ZEICHNUNGEN

Die begleitenden Zeichnungen sind umfasst, um ein weiteres Verständnis der Erfindung bereitzustellen und sind eingearbeitet in und stellen einen Teil dieser Beschreibung dar. Die Zeichnungen stellen exemplarische Ausführungsformen dar und dienen zusammen mit der Beschreibung zum Erklären der Grundlagen der Erfindung.
Fig. 1 ist eine Perspektivansicht eines Mundeinsatzes zur Anwendung mit einer Zusammensetzung gemäß einer bevorzugten Ausführungsform der vorliegenden Offenbarung.
Fig. 2 ist eine Detailansicht einer Innenfläche eines Mundeinsatzes zur Anwendung mit einer Zusammensetzung gemäß einer bevorzugten Ausführungsform der vorliegenden Offenbarung.
Fig. 3 ist ein Ablaufdiagramm zur Anwendung einer Zusammensetzung gemäß einer bevorzugten Ausführungsform bzw. einer Verwendung gemäß der vorliegenden Offenbarung.

### DETAILLIERTE BESCHREIBUNG UND BEVORZUGTE AUSFÜHRUNGSFORMEN DER ERFINDUNG

Die erfindungsgemäße Zahnreinigungs- und Zahnpflegezusammensetzung umfasst
- 20 bis 40 Gew.-% wenigstens eines Putzkörpers,
- 30 bis 45 Gew.-% wenigstens eines Feuchthaltemittels wobei das Feuchthaltemittel ausgewählt ist aus der Gruppe, bestehend aus Glycerin, Sorbitol, Xylit und beliebigen Mischungen davon,
- 0,8 bis 3,5 Gew.-% wenigstens eines Viskositätsreglers und Schaumbildners,
- 0,5 bis 1,8 Gew.-% wenigstens eines Schaumstabilisators,
- 3,5 bis 8 Gew.-% wenigstens eines Wirkstoff, insbesondere wenigstens eines Fluorid-Wirkstoffs, wenigstens eines Mittels zur Zahnfleischpflege und/oder wenigstens eines desinfizierenden Bestandteils, beispielsweise eines Bakterizides,
- 0 bis 1,0 Gew.-%, vorzugsweise 0,3 bis 1,0 Gew.-%, wenigstens eines Farbpigments,
- 0 bis 1,5 Gew.-%, vorzugsweise 0,5 bis 1,5 Gew%, wenigstens eines Aromastoffs, und
- Lösungsmittel ad 100 Gew.-%.

Die erfindungsgemäße Zusammensetzung weist eine Viskosität im Bereich von 50 - 10.000 m Pa s bei 20°C (1013,25 mbar, SVM™ 3001 Stabinger Viskosimeter™ von Anton Paar nach ASTM D7042) auf. Vorzugsweise liegt die Viskosität in einem Bereich von 100 - 8.000 m Pa s bei 20°C (1013,25 mbar, SVM™ 3001 Stabinger Viskosimeter™ von Anton Paar nach ASTM D7042), noch mehr bevorzugt bei 500 - 6.000 m Pa s bei 20°C (1013,25 mbar, SVM™ 3001 Stabinger Viskosimeter™ von Anton Paar nach ASTM D7042), höchst bevorzugt in einem Bereich von 1.500 bis 3.500 m Pa s bei 20 °C (1013,25 mbar, SVM™ 3001 Stabinger Viskosimeter™ von Anton Paar nach ASTM D7042).

Durch die Kombination der vorstehend genannten wesentlichen Bestandteile wird eine Zusammensetzung ermöglicht, die besonders zur gleichmäßigen, sparsamen und einfachen Applikation auf den oder die Mundeinsätze der Zahnputzvorrichtung geeignet ist.

Die erfindungsgemäße Zusammensetzung ist zur Reinigung und Pflege der Zähne, der Zahnhälse und des Zahnfleisches und insbesondere zur Vorbeugung und/oder Behandlung von Zahn- und Zahnfleischerkrankungen, insbesondere von Karies, Parodontose und Plaque, geeignet.

Die Inhalts- und Hilfs- oder Zusatzstoffe der Zusammensetzung zur Mund- und/oder Zahnreinigung und -pflege sind grundsätzlich die, wie sich auch für bereits bekannte Zahnreinigungsmittel, wie etwa Zahncremes, Zahnreinigungsgelen und dergleichen, eingesetzt werden. Dennoch haben sich bestimmte Inhalts- und Hilfs- oder Zusatzstoffe und/oder deren Kombination miteinander als besonders vorteilhaft herausgestellt, um eine Zusammensetzung bereitzustellen, die gleichmäßig, sparsam und einfach auf den Mundeinsatz oder die Mundeinsätze der Zahnputzvorrichtung zum gleichzeitigen Reinigen von mehrerer, vorzugsweise aller Zähne eines Benutzers, appliziert werden kann. Darüber hinaus stellt die Zusammensetzung sicher, dass genügend Wirkstoff appliziert wird, aber gleichzeitig eine Überdosierung vermieden wird.

### Putzkörper:

Der Putzkörper, der auch als Abrasivmittel oder Poliermittel bezeichnet werden kann, ist im Grunde ein Schleifmittel und ermöglicht eine effektive Entfernung von Belägen beim Zähneputzen. Als Putzkörper werden in der Regel wasserunlösliche anorganische Stoffe eingesetzt, die den Zahnbeleg mechanisch entfernen, ohne den Zahnschmelz oder das Dentin zu schädigen. Der Putzkörper kann eine Substanz oder eine Mischung von verschiedenen Substanzen enthalten. Erfindungsgemäß besonders bevorzugte Putzkörper sind Kieselsäure (Hydrated Silica), Silica Dimethyl Silyate oder Aluminiumoxid. Diese können einzeln oder in beliebigen Mischungen davon vorliegen. Weitere, im Stand der Technik bekannte Putzkörpersubstanzen können zusätzlich in diesem bevorzugten Putzkörper umfasst sein.

Der wenigstens eine Putzkörper ist mit einem Anteil von 20 bis 40 Gew.-%, bezogen auf die Gesamtzusammensetzung, in der erfindungsgemäßen Zusammensetzung umfasst. Vorzugsweise beträgt der Anteil des wenigstens einen Putzkörpers 25 bis 35 Gew.-%, noch mehr bevorzugt, 27 bis 33 Gew.-%, jeweils bezogen auf die Gesamtzusammensetzung.

### Feuchthaltemittel:

Feuchthaltemittel dienen zum einen dazu, ein Austrocknen der Zusammensetzung zu verhindern. Sie können aber auch als Konsistenzgeber und als Kältestabilisator fungieren. Pharmazeutisch und/oder kosmetisch akzeptable Feuchthaltemittel sind dem Fachmann grundsätzlich bekannt. Erfindungsgemäß umfasst die Zusammensetzung als Feuchthaltemittel Glycerin, Sorbitol oder Xylit oder beliebige Mischungen von diesen Feuchthaltemitteln. Weitere, im Stand der Technik bekannte Feuchthaltemittel können zusätzlich in diesen Feuchthaltemitteln umfasst sein.

Das wenigstens eine Feuchthaltemittel ist mit einem Anteil von 30 bis 45 Gew.-%, bezogen auf die Gesamtzusammensetzung, in der erfindungsgemäßen Zusammensetzung umfasst. Vorzugweise beträgt der Anteil des wenigstens einen Feuchthaltemittels 35 bis 43 Gew.-%, noch mehr bevorzugt 38 bis 42 Gew.-%, jeweils bezogen auf die Gesamtzusammensetzung.

### Viskositätsregler und Schaumbildner:

Pharmazeutisch und/oder kosmetisch akzeptable Viskositätsregler und Schaumbildner sind dem Fachmann grundsätzlich bekannt. Es können beispielsweise anionische oder kationische Tenside hierfür verwendet werden. Ebenso seien beispielshaft 1-Hexadecanol, 1-Octadecanol, Polyacrylsäure, Polyethylenglykol, pyrogene Kieselsäure (fumed Silica) oder beliebige Mischungen davon genannt. In einer erfindungsgemäß bevorzugten Ausführungsform umfasst die Zusammensetzung als Viskositätsregler und Schaumbildner Natriumlaurylsulfat und/oder Natriumlaurylsarkosinat. Besonders bevorzugt wird Cocamidopropyl Betaine als Viskositätsregler und Schaumbildner eingesetzt. Eine weitere Ausführungsform enthält eine Mischung aus Natriumlaurylsulfat und/oder Natriumlaurylsarkosinat und/oder Cocamidopropyl Betaine als Viskositätsregler und Schaumbildner. Diesen Verbindungen kommt in der erfindungsgemäßen Zusammensetzung sowohl eine Funktion bei der Bildung von Schaum als auch bei der Regelung der Viskosität der Zusammensetzung zu.

Der Anteil des wenigstens einen Viskositätsreglers und Schaumbildners in der erfindungsgemäßen Zusammensetzung beträgt 0,8 bis 3,5 Gew.-%, bezogen auf die Gesamtzusammensetzung. Vorzugweise beträgt der Anteil des wenigstens einen Viskositätsreglers und Schaumbildners 1,5 bis 2,5 Gew.-%, bezogen auf die Gesamtzusammensetzung.

### Schaumstabilisatoren:

In einer erfindungsgemäß bevorzugten Ausführungsform umfasst die Zusammensetzung als Schaumstabilisator Carboxymethylcellulose und/oder Hydroxyethylcellulose. Besonders bevorzugt wird Xanthan Gum als Schaumstabilisator eingesetzt. Eine weitere Ausführungsform enthält eine Mischung aus Carboxymethylcellulose und/oder Hydroxyethylcellulose und/oder Xanthan Gum als Schaumstabilisator.

Der Anteil des wenigstens einen Schaumstabilisators in der erfindungsgemäßen Zusammensetzung beträgt 0,5 bis 1,8 Gew.-%, bezogen auf die Gesamtzusammensetzung. Vorzugweise beträgt der Anteil des wenigstens einen Schaumstabilisators 0,8 bis 1,6 Gew.-%, noch mehr bevorzugt 1 bis 1,4 Gew.-%, jeweils bezogen auf die Gesamtzusammensetzung.

### Wirkstoffe:

Neben dem Putzkörper umfasst die erfindungsgemäße Zusammensetzung mindestens einen weiteren Wirkstoff. Der oder die weiteren Wirkstoffe umfassen wenigstens einen Fluorid-Wirkstoff, wenigstens ein Mittel zur Zahnfleischpflege, wenigstens ein Mittel zur Reduktion von Zahnbelägen und/oder wenigstens ein entzündungshemmendes Mittel und wenigstens einen desinfizierenden Bestandteil, wie beispielsweise Bakterizide. Die weiteren Wirkstoffe können einzeln oder in beliebigen Kombinationen in der erfindungsgemäßen Zusammensetzung umfasst sein.

Der Anteil des wenigstens einen oder der weiteren Wirkstoffe in der erfindungsgemäßen Zusammensetzung beträgt 3,5 bis 8 Gew.-%, bezogen auf die Gesamtzusammensetzung. Vorzugweise beträgt der Anteil des wenigstens einen oder der Wirkstoffe 4 bis 7,5 Gew.-%, noch mehr bevorzugt 6 bis 7 Gew.-%, jeweils bezogen auf die Gesamtzusammensetzung.

Als Fluorid-Wirkstoff wird besonders bevorzugt Aminfluorid oder Zinnfluorid oder eine Mischung davon verwendet. Der oder die Fluorid-Wirkstoffe sind vorzugsweise mit einem Anteil von 0,05 bis 0,3 Gew.-%, bezogen auf die Gesamtzusammensetzung enthalten. Noch mehr bevorzugt beträgt der Anteil 0,1 bis 0,2 Gew.-%.

Als Mittel zur Zahnfleischpflege wird besonders bevorzugt Allantoin, Panthenol, Extrakte aus Arnika, Myrrhe, Ratanhia, Salbei, Echinacea, Kamille, oder Aloe oder eine beliebige Mischung von diesen Substanzen verwendet. Das Mittel zur Zahnfleischpflege ist, falls vorhanden, vorzugsweise mit einem Anteil von 0,2 bis 3 Gew.-%, noch mehr bevorzugt mit einem Anteil von 0,5 bis 2 Gew.-%, bezogen auf die Gesamtzusammensetzung enthalten.

Als Mittel zur Reduktion von Zahnbelägen wird besonders bevorzugt Chlorhexidindigluconat, Zinkaspartat oder Arginin verwendet. Gemäß einer weiteren erfindungsgemäßen Ausführungsform enthält das Mittel zur Reduktion von Zahnbelägen eine beliebige Mischung aus Chlorhexidindigluconat, Zinkaspartat und/oder Arginin.

Das Mittel zur Reduktion von Zahnbelägen ist, falls vorhanden, vorzugsweise mit einem Anteil von 0,2 bis 3 Gew.-%, noch mehr bevorzugt mit einem Anteil von 0,5 bis 2 Gew.-%, bezogen auf die Gesamtzusammensetzung enthalten.

Weitere Wirkstoffe umfassen auch entzündungshemmende Mittel und desinfizierende Bestandteile, wie Bakterizide. Sie sind, falls vorhanden, vorzugsweise mit einem Anteil von 0,1 bis 3 Gew.-%, noch mehr bevorzugt mit einem Anteil von 0,2 bis 2 Gew.-%, bezogen auf die Gesamtzusammensetzung enthalten.

### Lösungsmittel:

Die erfindungsgemäße Zusammensetzung umfasst weiterhin wenigstens ein Lösungsmittel. Als Lösungsmittel kann beispielsweise Wasser, vorzugsweise Wasser welches für kosmetische oder pharmazeutische Formulierungen geeignet ist, eingesetzt werden. Weitere pharmazeutisch und/oder kosmetisch akzeptable Lösungsmittel sind dem Fachmann bekannt.

Der Anteil des wenigstens einen Lösungsmittels ist der Rest auf 100 Gew.-% (ad. 100 Gew.-%), bezogen auf die Gesamtzusammensetzung. Das heißt die Gew.-%-Anteile der wesentlichen und gegebenenfalls vorhandenen optionalen Bestandteile der Zusammensetzung sind immer so gewählt, dass sich in der Summe 100 Gew.-% ergeben.

Von besonderer Bedeutung für die Eignung der erfindungsgemäßen Zusammensetzung zu deren Applikation mittels einer Zahnputzvorrichtung für die gleichzeitige Reinigung mehrerer Zähne sind dabei das wenigstens eine Feuchthaltemittel, der wenigstens eine Viskositätsregler und Schaumbildner und der wenigstens eine Schaumstabilisator.

Zum Erreichen der gewünschten Viskosität ist insbesondere die Kombination aus wenigstens einem Feuchthaltemittel und wenigstens einem Viskositätsregler entscheidend, wobei der oder die Viskositätsregler gleichzeitig auch verantwortlich für die Schaumbildung sind.

Diese Hilfs- oder Zusatzstoffe ermöglichen die Bereitstellung einer niedrigviskosen Zusammensetzung, die in einer zweiten Stufe in ein Aerosol oder einen Schaum, vorzugsweise ein Schaumspray, überführt und gleichmäßig auf den oder die Mundeinsätze verteilt werden kann. Der gebildete Schaum ist für einen ausreichenden Zeitraum stabil und haftet damit für einen ausreichenden Zeitraum an den Mundeinsätzen. Ein Abfließen der Zusammensetzung bzw. des daraus gebildeten Schaums oder der Schaumtropfen oder der Aerosoltropfen vor dem Einsetzen in den Mund kann somit vermieden werden.

Optionale Bestandteile der Zusammensetzung sind:

### Farbpigmente:

Die erfindungsgemäße Zusammensetzung kann weiterhin ein oder mehrere Farbpigmente umfassen. Pharmazeutisch und/oder kosmetisch akzeptable Farbpigmente sind dem Fachmann bekannt. Diese können einzeln oder in beliebigen Mischungen davon eingesetzt werden. Vorzugsweise wird als Farbpigment Chlorophyllin (C.I. 75810), gegebenenfalls auch in Mischung mit weiteren Farbpigmenten eingesetzt. Der Anteil an Farbpigment, sei es eine Farbpigment oder eine Mischung aus mehreren Farbpigmenten, beträgt vorzugsweise 0 bis 1,0 Gew.-%, vorzugsweise 0,2 bis 1,0 Gew.-%, bezogen auf die Gesamtzusammensetzung. Noch mehr bevorzugt beträgt der Bereich 0,3 bis 0,8 Gew.-%.

### Aromastoffe:

Die erfindungsgemäße Zusammensetzung kann ferner einen oder mehrere Aromastoffe umfassen. Besonders bevorzugt umfasst der Aromastoff Anethol, Menthol oder Limonen oder eine beliebige Kombination von diesen bevorzugten Aromastoffen. Der Anteil an Aromastoffen, sei es ein Aromastoff oder eine Mischung aus mehreren Aromastoffen, beträgt vorzugsweise 0 bis 1,5 Gew.-%, vorzugsweise 0,2 bis 1,3 Gew.-%, bezogen auf die Gesamtzusammensetzung. Noch mehr bevorzugt beträgt der Anteil 0,5 bis 1,1 Gew.-%.

Als weitere optionale Bestandteile kann die erfindungsgemäße Zusammensetzung umfassen wenigstens ein Bindemittel, wenigstens einen Lösungsvermittler, wenigstens ein Netzmittel, wenigstens einen Süßstoff, wenigstens ein Bleichmittel und/oder wenigstens einen pH-Regler. Die erfindungsgemäße Zusammensetzung kann als optionalen Bestandteil auch einen oder mehrere weitere Stabilisatoren, wie beispielsweise Natriumgluconat (INCI: Sodium Gluconate) umfassen. Dieser wenigstens eine weitere Stabilisator kann eine Entmischung der Emulsion verhindern und/oder eine Zersetzung der anderen Bestandteile der Zusammensetzung, beispielsweise der Wirkstoffe, verhindern. Natriumgluconat verhindert beispielsweise nicht nur eine Entmischung der erfindungsgemäßen Zusammensetzung, es verhindert darüber hinaus auch eine oxidative Zersetzung der weiteren Bestandteile, insbesondere der Wirkstoffe, der Zusammensetzung. Diese optionalen Bestandteile können einzeln oder in beliebigen Kombinationen miteinander in der erfindungsgemäßen Zusammensetzung umfasst sein.

Alle Bestandteile der Zusammensetzung sind in einem Reinheitsgrad, der für Zahnpflegezusammensetzungen geeignet und notwendig ist. Werden für bestimmte Hilfs- oder Zusatzstoffe hierin keine Angaben zu deren Gew.-%-Anteil gemacht, bedeutet dies, dass der Fachmann die bevorzugten Bereiche kennt oder einfach ermitteln kann.

Ebenfalls im Umfang der vorliegenden Erfindung mit umfasst sind solche Zusammensetzungen, die, mit Ausnahme von üblichen Verunreinigungen, nur aus den vorstehend genannten wesentlichen, und gegebenenfalls einer oder mehreren der optionalen Bestandteile, bestehen.

Die Angabe Gew.-%, wie in der vorliegenden Patentanmeldung verwendet, bedeutet, sofern nichts anderes angegeben wird, dass der Anteil auf das Gesamtgewicht der Zusammensetzung bezogen ist.

Die erfindungsgemäße Zusammensetzung weist eine dynamische Viskosität im Bereich von 50 - 10.000 m Pa s bei 20°C (1013,25 mbar, SVM™ 3001 Stabinger Viskosimeter™ von Anton Paar nach ASTM D7042) auf. Vorzugsweise liegt die Viskosität in einem Bereich von 100 - 8.000 m Pa s bei 20°C, noch mehr bevorzugt bei 500 - 6.000 m Pa s bei 20°C, höchst bevorzugt in einem Bereich von 1.500 bis 3.500 m Pa s bei 20 °C (jeweils gemessen bei 1013,25 mbar, SVM™ 3001 Stabinger Viskosimeter™ von Anton Paar nach ASTM D7042). Der gewählte Viskositätsbereich stellt sicher, dass die erfindungsgemäße Zusammensetzung gleichmäßig und einfach auf den Mundeinsatz oder die Mundeinsätze der Zahnputzvorrichtung appliziert werden kann und auch genügend lange an diesem/diesen haftet.

Eine besonders bevorzugte erfindungsgemäße Zusammensetzung umfasst 20 bis 40 Gew.-% Silica Dimethyl Silyate, Kieselsäure (Hydrated Silica) oder einer Mischung von beiden, 30 bis 45 Gew.-% Sorbitol oder Glycerin oder Xylit oder einer beliebigen Mischung davon, 0,8 bis 3,5 Gew.-% Natriumlaurylsulfat und/oder Cocamidopropyl Betaine, 0,5 bis 1,8 Gew.-% Hydroxyethylcellulose, 3,5 bis 8 Gew.-%, vorzugsweise 4 bis 8 Gew.-% eines Wirkstoffs, ausgewählt aus der Gruppe, bestehend aus wenigstens einem Fluorid-Wirkstoff, wenigstens einem Mittels zur Zahnfleischpflege, wenigstens einem Mittel zur Reduktion von Zahnbelägen, wenigstens einem desinfizierenden Bestandteil und beliebigen Mischungen davon, sowie ein Lösungsmittel, vorzugsweise Wasser ad 100 Gew.-%.

Diese besonders bevorzugte Zusammensetzung kann entweder, abgesehen von üblichen Verunreinigungen, nur aus diesen Bestandteilen bestehen, oder sie kann darüber hinaus noch 0,3 bis 1,0 Gew.-% Chlorophyllin und/oder 0,5 bis 1,5 Gew% Anethol oder Menthol oder Limonen oder eine beliebige Mischung davon umfassen. Darüber hinaus können in der Zusammensetzung noch umfasst sein Saccharin oder ein anderer Süßstoff, ein pH-Regler, PEG-40 hydriertes Rizinusöl (INCI: PEG-40 Hydrogenated Castor Oil) und/oder Natriumgluconat (INCI: Sodium Gluconate). Gemäß einer weiteren erfindungsgemäßen Ausführungsform besteht die Zusammensetzung, abgesehen von üblichen Verunreinigungen, nur aus den genannten wesentlichen und einem oder mehrerer der genannten optionalen Bestandteile.

In einer erfindungsgemäßen Ausführungsform enthält die Zahnpflegezusammensetzung entweder Fluoride, als weiteren Wirkstoff, oder Hydroxylapatit, jedoch nie beide Bestandteile gleichzeitig.

Ebenso ist es, wie bereits vorstehend ausgeführt, vorteilhaft, wenn die Zusammensetzung ausgehend von der niederviskosen Mischung in einem zweiten Stadium bei der Applikation in einen Schaum, vorzugsweise in ein Schaumspray, mit einer bestimmten Lebensdauer oder in ein Aerosol überführt wird. Deshalb kommt dem oder den Schaumbildnern und/oder dem oder den Schaumstabilisatoren ebenfalls eine besondere Bedeutung zu. Erfindungsgemäß besonders bevorzugt sind hierbei auch Cocamidopropyl, Natriumlaurylsulfat, Trinatriumphosphat, Natriummethylcococyltaurat, einzeln oder in beliebigen Mischungen davon.

In einem weiteren Aspekt betrifft die vorliegende Erfindung daher die vorstehend im Detail beschriebene Zusammensetzung in der Form eines Schaums, vorzugsweise eines Schaumsprays, oder in der Form eines Aerosols. Bezüglich der wesentlichen und optionalen Inhalts- und Hilfs- oder Zusatzstoffe sowie deren Anteilsbereiche und bevorzugten Anteilsbereiche wird ausdrücklich auf die vorstehenden Ausführungen im Zusammenhang mit der Zusammensetzung Bezug genommen. Die Darreichungsform als Schaum, vorzugsweise als Schaumspray, oder Aerosol ermöglicht im Besonderen eine gleichmäßige, sparsame und einfache Applikation der erfindungsgemäßen Zusammensetzung auf den Mundeinsatz oder die Mundeinsätze der Zahnputzvorrichtung zum gleichzeitigen Putzen mehrerer, vorzugsweise aller Zähne eines Benutzers. Die Zusammensetzung haftet so besonders gut und gleichmäßig an dem oder den Mundeinsätzen.

In einem weiteren, bevorzugten Aspekt betrifft die Erfindung auch die vorstehend beschriebene Zusammensetzung abgefüllt in einen Behälter bzw. Spender bzw. eine Vorrichtung, der/die es ermöglicht, dass sie als Schaum, vorzugsweise als Schaumspray, oder als Aerosol dargereicht oder ausgebildet werden kann. Der Behälter umfasst ein Fördersystem zum Befördern der Zusammensetzung nach außen, ein Ventil und eine Schaumdüse. In einer erfindungsgemäßen Ausführungsform befördert das Fördersystem die Zusammensetzung mittels eines Treibgases, Druckluft oder über ein Pumpsystem aus dem Behälter. Die Austrittsöffnung des Behälters, beispielsweise die Schaumdüse, ist vorzugsweise derart ausgestaltet, dass eine gleichmäßige Verteilung der erfindungsgemäßen Zusammensetzung auf dem oder den Mundeinsätzen gewährleistet wird. Der Behälter kann ein Behälter mit zwei Kammern sein. In einem Innenbeutel befindet sich die erfindungsgemäße Zusammensetzung und in einer äußeren Kammer ein oder mehrere Treibgase.

Im Folgenden wird die Funktionsweise einer beispielhaften Schaum- oder Aerosolsprühvorrichtung beschrieben mit der die hierin beschriebene Zusammensetzung appliziert werden kann. Durch den Innendruck einer Spraydose wird der Inhalt genau dann freigesetzt, wenn man den Sprühkopf betätigt. Ein Teil des Treibmittels (Luft, Kohlendioxid, Distickstoffoxid, Dimethylether, Propan/Butan, Isopentan) ist gelöst in der Wirkstofflösung (der eigentlichen Zusammensetzung der vorliegenden Offenbarung) und der andere Teil liegt als Gas vor. Wird der Sprühknopf nun betätigt, treibt der gasförmige Anteil des Treibmittels, die Wirkstofflösung durch das Ventil nach außen. Jetzt verdampft das Treibmittel extrem schnell und die zurückbleibende Wirkstofflösung verteilt sich sehr fein und gleichmäßig.

Die niedrigviskose Zusammensetzung befindet sich in einer bevorzugten Ausführungsform in einer so genannten Bag-in-Can Dose. Mit dieser Aerosoltechnologie lassen sich auch dickflüssigere Produkte austragen. Hierbei werden Treibgas und Wirkstofflösung voneinander getrennt und liegen in zwei getrennten Kammern vor. Ein Beutel, beispielsweise ein Aluminiumbeutel, beinhaltet die Wirkstofflösung und dieser Beutel ist mit dem Ventil verbunden. Das Treibmittel umgibt den Beutel und liefert den notwendigen Druck. Bei der Betätigung des Sprühkopfes quetscht das Treibmittel nun Produkt aus dem Beutel. Die äußere Dose besteht vorzugsweise aus Weißblech oder Aluminium, weil diese Materialien stabil, leicht und sehr gut recycelbar sind.

Im Folgenden wird die Ventiltechnik einer bevorzugten Ausführungsform beschrieben. Auf der Oberseite der Spraydose wird ein offenes Plastikröhrchen mit einem Loch in der Seite befestigt. Ein Gummiring dichtet dieses seitliche Loch ab. An der unteren Seite des Röhrchens ist eine Feder aufgesteckt, die mit dem unteren Teil des Röhrchens in einem Plastikgehäuse sitzt. Der Gummiring liegt auf dem Rand der Dose auf. Der Ventilteller presst diese Anordnung so fest auf, dass sich nur noch das Röhrchen bewegen kann. Der Sprühkopf, der auf dem Plastikröhrchen steckt, bewegt nun das Röhrchen nach unten und gibt das Loch frei. Hierdurch kann nun Gas und die niedrigviskose Zusammensetzung austreten. Lässt der Druck auf den Sprühkopf nach, verschließt der Gummiring wieder das Loch im Röhrchen und die Dose ist wieder dicht.

Neben der niedrigviskose Zusammensetzung spielen bei einem Aerosol bzw. Schaum noch anderen technische Parameter wie
- das Verhältnis von Wirkstofflösung zu Treibmittel,
- die Art des Treibmittels,
- die Größe der Ventilöffnung und/oder
- die Größe der Sprühkopföffnung
   eine Rolle.

Als Treibgase zur Herstellung eines Schaums oder Aerosols werden in bevorzugten Ausführungsformen Dimethylether, Propan/Butan, Isopentan oder beliebige Mischungen davon verwandt. Der Anteil an Treibgas in der Zusammensetzung beträgt vorzugsweise zwischen 4 und 40 Gew.-%. Alternativ können auch Luft, Kohlendioxid, Distickstoffoxid oder beliebige Mischungen davon als Treibmittel eingesetzt werden. Eine Alternative zur Herstellung eines Schaums oder eines Aerosols aus der Zusammensetzung stellt die Verwendung eines manuellen oder elektrischen Pumpsystems dar. Wird die Pumpe betätigt, entsteht ein Überdruck zwischen einem äußeren und einem inneren Behälter, der nach Betätigung des Ventils das Aerosol bzw. den Schaum bildet. Die Anwendung der Zusammensetzung als Schaum ist besonders bevorzugt.

Die Größe der Ventilöffnung und die Größe der Sprühkopföffnung steht in direkter Korrelation zur Tröpfchengröße des Aerosols oder des Schaums. Für den Ventilkopf liegt sie vorzugsweise zwischen 0,2 und 4 mm, für den Sprühkopf vorzugsweise zwischen 0,4 und 6 mm.

Eine niedrigviskose Mund-, Zahnpflege- und Zahnreinigungszusammensetzung, die die üblichen pharmazeutisch/kosmetisch akzeptablen Inhaltsstoffe und Zusatz- oder Hilfsstoffe enthält, mit einer Viskosität von 50 - 10.000 m Pa s (bei 20°C, 1013,25 mbar, SVM™ 3001 Stabinger Viskosimeter™ von Anton Paar nach ASTM D7042) kann in einem Verfahren zur Zahnreinigung und -pflege mittels einer Vibrationszahnputzvorrichtung für die gleichzeitige Reinigung mehrerer, vorzugsweise aller Zähne eines Benutzers verwendet werden, wobei die Zusammensetzung dargereicht wird durch Aufbringen der Zusammensetzung auf einen Mundeinsatz oder mehrere Mundeinsätze der Zahnputzvorrichtung.

Die Zusammensetzung ist insbesondere zur Verwendung in einem Verfahren zum Vorbeugen und/oder Behandeln von Erkrankungen der Zähne, der Zahnhälse und/oder des Zahnfleisches, insbesondere von Karies, Parodontose und Plaque, bestimmt.

Die Zusammensetzung kann als Schaum, noch mehr bevorzugt als Schaumspray, oder als Aerosol auf den Mundeinsatz oder auf die Mundeinsätze der Zahnputzvorrichtung appliziert werden.

Der Schaum kann, vorzugsweise als Schaumspray, oder das Aerosol kann mittels einem der vorstehend beschriebenen Behälter bereitgestellt werden.

Als für die offenbarte Verwendung besonders bevorzugte Zusammensetzungen haben sich insbesondere die vorstehend beschriebenen Zusammensetzungen herausgestellt. Diese stellen eine genügend hohe Wirkstoffmenge bereit und ermöglichen gleichmäßige, sparsame und einfache Applikation der Zusammensetzung auf den Mundeinsatz oder die Mundeinsätze.

Die Fig. 1 zeigt eine erste Ausführungsform eines Mundeinsatzes zur Anwendung mit einer Zusammensetzung gemäß einer bevorzugten Ausführungsform der vorliegenden Offenbarung. Insbesondere zeigt Fig. 1 eine perspektivische Ansicht eines einteiligen Mundeinsatzes 10. Die nicht sichtbare untere Struktur des Mundeinsatzes ist der oberen im Wesentlichen identisch, wobei anpassungsbedingte Formabweichungen möglich sind. Die Wandstärke der Wände 12 des Mundeinsatzes 10 beträgt typischerweise 1 bis 2,5 mm. Die äußere Begrenzung der Wände 12 verläuft in der Regel 1 bis 3 mm oberhalb des Zahnfleischrandes. Der Abstand zwischen den Reinigungsflächen, d.h. denjenigen Flächen des Mundeinsatzes 10, die beim Reinigungsvorgang den Zahnflächen des Benutzers zugewandt sind, und den Zahnoberflächen beträgt 0 bis 5 mm in Abhängigkeit von den gewählten Reinigungsstrukturen 14. Auf den Reinigungsflächen können Reinigungsstrukturen 14 vorgesehen sein.

An dem Mundeinsatz 10 der Ausführungsform in Fig.1 ist ein Kupplungselement 16 zur Verbindung des Mundeinsatzes 10 mit einer Antriebsvorrichtung (nicht gezeigt) angebracht. Die Antriebsvorrichtung ist grundsätzlich mit Antriebsvorrichtungen vergleichbar, wie sie aus dem Stand der Technik auf dem Gebiet der Schallzahnbürsten oder dergleichen bekannt sind. Die Kopplung mit der Antriebsvorrichtung erfolgt über eine passende Bohrung 24.

In Fig. 1 wird verdeutlicht, dass ein relativ genau an das Gebiss des Benutzers angepasster Mundeinsatz viele Ausbuchtungen (den Zähnen entsprechend) und Stege (den Zahnzwischenräumen und Kauflächen entsprechend) aufweist. Diese Unebenheiten erschweren die Nutzung mit einer herkömmlichen Zahncreme oder ähnlichen Präparaten deutlich. Entweder muss sehr viel Zahncreme aufgetragen werden, was die Anwendungskosten erhöht und Ressourcen verschwendet. Ein akkurates Verteilen einer zähflüssigen Zahncreme mit einer Bürste oder einem Spatel wäre außerdem sehr zeitaufwändig für den Anwender/Benutzer. Die Reinigungsstrukturen 14 (in Fig. 2 gezeigt und unten detailliert beschrieben) erzeugen zusätzlich feine Unebenheiten, die mit einer herkömmlichen Zahncreme kaum zu erreichen bzw. auszufüllen sind.

Fig. 2 zeigt eine Detailansicht von Reinigungsstrukturen 14 in Form von Reinigungselementen, die an der Oberfläche des Mundeinsatzes 10 aus Fig. 1 vorgesehen sein können. In diesem Fall handelt es sich jeweils um Reinigungselemente, die einstückig mit dem Mundeinsatz 10 an dessen Oberfläche gebildet sind. Im Bereich der Kauflächen sind in dieser Ausführungsform rautenförmige Reinigungselemente 28 vorgesehen, im Bereich der vorderen und hinteren Zahnflächen sind jeweils zylinderförmige Reinigungselemente 30 ausgebildet. Aufgrund der Rautenform sind die Reinigungselemente 28 härter als die zylinderförmigen Reinigungselemente 30, so dass im Bereich der Kauflächen eine intensivere Zahnputzwirkung erzielt wird. Selbstverständlich können die Reinigungselemente 30 an den vorderen und hinteren Zahnflächen verschieden ausgebildet sein. Ebenso könnten in jedem der in Fig. 2 gezeigten Bereiche, d. h. im Bereich der hinteren Zahnflächen, der Kauflächen und der vorderen Zahnflächen, auch andere Reinigungsstrukturen 14 vorgesehen sein, beispielsweise Gummierungsschichten und/oder Streifenbürsten.

Durch die variierende Formgebung der Reinigungsstrukturen wird die Nutzung herkömmlicher Zahncremes zusätzlich erschwert. Dringt eine bestimmte Zahncreme beispielsweise gut in den Raum zwischen den rautenförmigen Elementen 28 ein, so sind die Abstände zwischen den Borstenstrukturen 30 zu gering, um eine ausreichende und einfache Verteilung der Zahncreme zu ermögliche und bei unachtsamer Anwendung kann sich das Reinigungsergebnis verschlechtern.

Die Anwendung einer niedrigviskosen Zusammensetzung gemäß der vorliegenden Offenbarung ermöglicht das leichte und fehlerfreie Auftragen auf einen Mundeinsatz gemäß Fig.1 und 2. Durch das bevorzugte Auftragen der Zusammensetzung in Form eines Schaums, noch mehr bevorzugt in Form eines Schaumsprays, wird insbesondere eine verbesserte Haftung auf den Reinigungsflächen in Fig. 1 gewährleistet, die nicht gezeigt, also nach unten gerichtet sind.

Aerosole und Schäume sind eine Möglichkeit Pflegeprodukte und Kosmetika in einer geeigneten Applikationsform anzubieten. Aerosole und Schäume bieten den Vorteil
- einer langen Haltbarkeit,
- einer exakten Dosierung und einer sparsamen Anwendung,
- darüber hinaus können in den Spender keine Mikroorganismen eindringen, so dass auch
- unkonservierte Produkte möglich sind.

Ein Aerosol ist eine Lösung nicht gasförmiger Stoffe in einem Gas. Es entsteht erst durch Betätigung des Sprühkopfs und wird damit erst bei Bedarf und in der benötigten Menge hergestellt. Ein Schaum wird durch die Anordnung einer Schaumdüse im Sprühkopf einer geeigneten Spendervorrichtung erzeugt, wobei die in der Spendervorrichtung enthaltene Zusammensetzung zusätzlich Tenside oder ähnliche Vernetzungsmittel, sowie vorzugsweise Schaumstabilisatoren enthalten sollte, die einen ausreichend stabile Schaumerzeugung ermöglichen. In Abhängigkeit der speziellen Eigenschaften der in der Zusammensetzung enthaltenen oberflächenaktiven Moleküle entstehen Schaumbläschen mit unterschiedlicher Größe, Wandstärke und Lebensdauer. Grundsätzlich ist die Lebensdauer von flüssigen Schäumen begrenzt. Diese kann von der Viskosität der den Schaum bildenden Flüssigkeit, also der hierin beschriebenen Zusammensetzung, und/oder den in der Zusammensetzung enthaltenen Zusatz- und Hilfsstoffen abhängen. Ein Schaumspray ist eine Emissionsform, in der zunächst ein Schaum durch eine Schaumdüse gebildet wird, der dann noch in der Sprayvorrichtung durch einen Gasstrom zerrissen wird und somit kleinere Schaumtropfen auf die Zielfläche emittiert werden.

Im Bereich der Pflegeprodukte liegen Schäume und Schaumsprays als Haarspray, Deospray, Rasierschaum, Rasiergel, Bodymousse, Enthaarungsmousse, Haartönungsmousse, Schaumfestiger, Desinfektionsspray, Fußspray, Sonnenschutzschäume und Kühlspray vor.

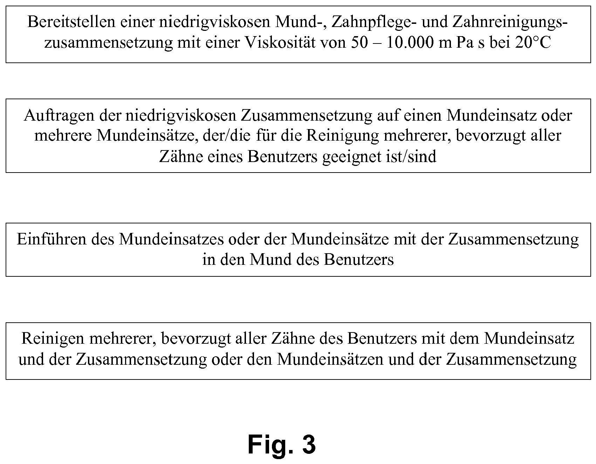

Das durch die vorliegende Offenbarung bereitgestellte Verfahren zum Reinigen und Pflegen der Zähne und/oder des Zahnfleisches eines Benutzers, insbesondere zum Vorbeugen und/oder Behandeln von Erkrankungen der Zähne, der Zahnhälse und/oder des Zahnfleisches mit einer niedrigviskosen Mund-, Zahnpflege- und Zahnreinigungszusammensetzung mit einer Viskosität von 50 - 10.000 m Pa s bei 20°C wird in Fig. 3 zusammengefasst. Das Verfahren gemäß der vorliegenden Offenbarung umfasst entsprechend das Bereitstellen einer niedrigviskosen Mund-, Zahnpflege- und Zahnreinigungszusammensetzung mit einer Viskosität von 50 - 10.000 m Pa s bei 20°C, das Auftragen der niedrigviskosen Zusammensetzung auf einen Mundeinsatz, der für die Reinigung mehrerer, bevorzugt aller Zähne eines Benutzers geeignet ist, das Einführen des Mundeinsatzes mit der Zusammensetzung in den Mund des Benutzers und das Reinigen mehrerer, bevorzugt aller Zähne des Benutzers mit dem Mundeinsatz und der Zusammensetzung.

Die bereitgestellte Zusammensetzung ist vorzugsweise die erfindungsgemäße Zusammensetzung, umfassend
- 20 bis 40 Gew.-% wenigstens eines Putzkörpers,
- 30 bis 45 Gew.-% wenigstens eines Feuchthaltemittels, wobei das Feuchthaltemittel ausgewählt ist : aus der Gruppe, bestehend aus Glycerin, Sorbitol, Xylit und beliebigen Mischungen davon,
- 0,8 bis 3,5 Gew.-% wenigstens eines Viskositätsreglers und Schaumbildners,
- 0,5 bis 1,8 Gew.-% wenigstens eines Schaumstabilisators,
- 3,5 bis 8 Gew.-% wenigstens eines Wirkstoffs, insbesondere wenigstens eines Fluorid-Wirkstoffs, wenigstens eines Mittels zur Zahnfleischpflege und/oder wenigstens eines desinfizierenden Bestandteils, beispielsweise eines Bakterizides,
- 0 bis 1,0 Gew.-%, vorzugsweise 0,3 bis 1,0 Gew.-%, wenigstens eines Farbpigments,
- 0 bis 1,5 Gew.-%, vorzugsweise 0,5 bis 1,5 Gew%, wenigstens eines Aromastoffs, und
- Lösungsmittel ad 100 Gew.-%.

Die Zusammensetzung weist eine Viskosität im Bereich von 50 - 10.000 m Pa s bei 20°C (1013,25 mbar, SVM™ 3001 Stabinger Viskosimeter™ von Anton Paar nach ASTM D7042) auf. Vorzugsweise liegt die Viskosität in einem Bereich von 100 - 8.000 m Pa s, noch mehr bevorzugt bei 500 - 6.000 m Pa s, höchst bevorzugt in einem Bereich von 1.500 bis 3.500 m Pa s (ebenfalls jeweils gemessen bei 20°C, 1013,25 mbar, SVM™ 3001 Stabinger Viskosimeter™ von Anton Paar nach ASTM D7042).

Bezüglich der bevorzugten wesentlichen und optionalen Inhalts- und Hilfs- oder Zusatzstoffe sowie deren bevorzugter Anteilsbereiche wird ausdrücklich auf die vorstehenden Ausführungen im Zusammenhang mit der Zusammensetzung als solches Bezug genommen.

Zum Reinigen der mehreren, vorzugsweise aller Zähne des Benutzers wird der Mundeinsatz bzw. werden die Mundeinsätze im Mund des Benutzers bewegt. Vorzugsweise wird der Mundeinsatz bzw. werden die Mundeinsätze im Mund des Benutzers durch einen Motor zu Schwingungen mit einer Frequenz im Bereich von 20 bis 700 Hz, bevorzugt im Bereich von 25 bis 600 Hz angeregt.

Das gleichzeitige Reinigen der mehreren, vorzugsweise aller Zähne des Benutzers, dauert vorzugsweise zwischen 2 Sekunden und 5 Minuten, noch bevorzugter liegt die Zahnputzdauer in einem Bereich von 3 Sekunden bis zu 4 Minuten. Alternativ liegt die Obergrenze der Putzdauer bei 2 Minuten, 1,5 Minuten oder 1 Minute.

Das offenbarte Verfahren zum Reinigen und Pflegen der Zähne und/oder des Zahnfleisches sollte vorzugsweise mindestens zweimal am Tag, noch mehr bevorzugt zwei bis viermal pro Tag, beispielsweise zweimal oder dreimal pro Tag, durchgeführt werden.

### Messmethoden:

Die Bestimmung der Viskosität erfolgt mit einem hochpräzisen Rotationsviskosimeter mit integrierter Dichtemesszelle (SVM™ 3001 Stabinger Viskosimeter™ von Anton Paar) gemäß der Norm ASTM D 7042. Das Anton Paar Viskosimeter SVM 3001 arbeitet nach dem Stabinger Messprinzip. Es basiert auf dem Couette-Prinzip, mit integrierter Dichtemesszelle. In einem einzigen Messzyklus können die dynamische Viskosität, Dichte, kinematische Viskosität und der Viskositätsindex bestimmt werden. Ein mit konstanter Drehzahl rotierendes Rohr wird mit der Probe gefüllt, in welcher sich der Messrotor, der langsamer als das Außenrohr rotiert, befindet. Es werden Drehmoment und Drehzahl bestimmt.

Das SVM™ 3001 Stabinger Viskosimeter™ von Anton Paar basiert auf dem Couette-Prinzip, mit integrierter Dichtemesszelle. In einem einzigen Messzyklus können die dynamische Viskosität, Dichte, kinematische Viskosität und der Viskositätsindex bestimmt werden. Die Messzellenkombination deckt den gesamten Messbereich für Viskosität, Dichte und Temperatur ab. Der Messbereich für Viskosität beträgt 0,2 bis 30 000 mm²/s; die simultane Dichte-Bestimmung erfolgt gemäß ASTM D4052 und ISO 12185. Es kann in einem Temperaturbereich von -60 ° bis +135 ° C betrieben werden.

Die Messungen erfolgen im newtonschen Bereich der Proben. Falls nicht anderes angegeben, gelten alle Viskositätsangaben bei 20 °C und Normaldruck von 0,1 MPa (1013,25 mbar). Bestimmt wird die Viskosität der flüssigen Zusammensetzung, also zu einem Zeitpunkt bevor die Zusammensetzung gemäß einer bevorzugten Ausführungsform in einen Schaum oder ein Aerosol überführt wird.

### BEISPIEL

### Beispiel 1 (Zahnpflegezusammensetzung)

| | | |
|---|---|---|
| Putzkörper | Silica Dimethyl Silylate Kieselsäure (Hydrated Silica) | 30 Gew.-% |
| Feuchthaltemittel | Sorbitol, Glycerin, Xylit | 40 Gew.-% |
| Viskositätsregelung und | Natriumlaurylsulfat | 0,8 Gew.-% |
| Schäumer | Cocamidopropyl Betaine | 1,1 Gew.-% |
| Schaumstabilisator | Hydroxyethylcellulose | 1 Gew.-% |
| Fluorid- Wirkstoffe | Olaflur Aminfluorid | 0,15 Gew.-% |
| Zahnfleischpflege | Allantoin, Azulen, Bisabolol, Carbamid, Panthenol | 2 Gew.-% |
| Reduktion von Zahnbelägen | Zinkaspartat, Arginin | 2 Gew.-% |
| Farbpigmente | Chlorophyllin (C.I.75810) | 0,5 Gew.-% |
| Aroma, Aromastoffe | Anethol, Menthol, Limonene, | 1 Gew.-% |
| Stabilisator | Natriumgluconat (Sodium Gluconate) | 1,2 Gew.-% |
| Netzmittel | PEG-3 Tallow | 3 Gew.-% |
| Lösungsvermittler | PEG-40 hydriertes Rizinusöl (PEG-40 Hydrogenated Castor Oil) | 1 Gew.-% |
| Süßstoff | Saccharin | 0,5 Gew.-% |
| Hilfsstoffe für pH Wert (5 bis 7,5) | Salzsäure (Hydrochloric Acid), Kaliumhydroxid (Potassium Hydroxide) | 1 Gew.-% |
| Lösungsmittel | Wasser (Aqua) | ad 100 Gew.-% |

Die vorstehend genannte, beispielhafte Zusammensetzung kann in den Mundstücken manuell durch Aufsprühen als Schaum oder Aerosol in die Mundstücke verteilt werden. Sie ist dadurch sehr gleichmäßig. Die Zusammensetzung haftet gut an den Mundstücken. Die Anhaftung (Adhäsion) ist messbar mit einem Tensiometer.

## Patentansprüche

1. Niedrigviskose Mund-, Zahnpflege- und Zahnreinigungszusammensetzung umfassend:
- 20 bis 40 Gew.-% wenigstens eines Putzkörpers,
- 30 bis 45 Gew.-% wenigstens eines Feuchthaltemittels, wobei das Feuchthaltemittel ausgewählt ist aus der Gruppe, bestehend aus Glycerin, Sorbitol, Xylit und beliebigen Mischungen davon,
- 0,8 bis 3,5 Gew.-% wenigstens eines Viskositätsreglers und Schaumbildners,
- 0,5 bis 1,8 Gew.-% wenigstens eines Schaumstabilisators,
- 3,5 bis 8 Gew.-% wenigstens eines Wirkstoff,
- 0 bis 1,0 Gew.-% wenigstens eines Farbpigments,
- 0 bis 1,5 Gew.-% wenigstens eines Aromastoffs, und
- Lösungsmittel ad 100 Gew.-%,
wobei die Zusammensetzung einer Viskosität im Bereich von 50 - 10.000 m Pa s, gemessen bei 20°C, 1013,25 mbar, mit einem SVM™ 3001 Stabinger Viskosimeter™ von Anton Paar nach ASTM D7042, aufweist.

2. Niedrigviskose Mund-, Zahnpflege- und Zahnreinigungszusammensetzung nach Anspruch 1, wobei der Anteil des wenigstens einen Putzkörpers an der Zusammensetzung 20 bis 33 Gew.-% beträgt.

3. Niedrigviskose Mund-, Zahnpflege- und Zahnreinigungszusammensetzung nach Anspruch 1 oder Anspruch 2, wobei der wenigstens eines Wirkstoff wenigstens ein Fluorid-Wirkstoffs, wenigstens ein Mittel zur Zahnfleischpflege und/oder wenigstens ein desinfizierender Bestandteil ist.

4. Niedrigviskose Mund-, Zahnpflege- und Zahnreinigungszusammensetzung nach Anspruch 3, wobei der desinfizierende Bestandteil ein Bakterizid ist.

5. Niedrigviskose Mund-, Zahnpflege- und Zahnreinigungszusammensetzung nach einem der vorhergehenden Ansprüchen, wobei der Anteil des wenigstens einen Farbpigments an der Zusammensetzung 0,3 bis 1,0 Gew.-% beträgt.

6. Niedrigviskose Mund-, Zahnpflege- und Zahnreinigungszusammensetzung nach einem der vorhergehenden Ansprüchen, wobei der Anteil des wenigstens einen Aromastoffs an der Zusammensetzung 0,5 bis 1,5 Gew.-% beträgt.

7. Niedrigviskose Mund-, Zahnpflege- und Zahnreinigungszusammensetzung nach einem der vorhergehenden Ansprüche, wobei das Lösungsmittel Wasser ist.

8. Schaum erhältlich aus der aus der Zusammensetzung gemäß einem der Ansprüche 1 bis 7.

9. Behälter, umfassend
- die Zusammensetzung nach einem der Ansprüche 1 bis 7,
- ein Fördersystem zum Befördern der Zusammensetzung nach außen,
- ein Ventil, und
- eine Schaumdüse.

10. Behälter nach Anspruch 9, wobei das Fördersystem die Zusammensetzung mittels eines Treibgases, Druckluft oder über ein Pumpsystem befördert.

## Claims

1. A low viscosity oral, dental care and dental cleansing composition comprising:
- 20 to 40% by weight of at least one cleaning agent,
- 30 to 45% by weight of at least one humectant, wherein the humectant is selected from the group consisting of glycerin, sorbitol, xylitol and any mixtures thereof,
- 0.8 to 3.5% by weight of at least one viscosity regulator and foaming agent,
- 0.5 to 1.8% by weight of at least one foam stabiliser,
- 3.5 to 8 % by weight of at least one active substance,
- 0 to 1.0% by weight of at least one colour pigment,
- 0 to 1.5% by weight of at least one flavouring agent, and
- solvent add up to 100% by weight,
wherein the composition has a viscosity in the range of 50 - 10,000 m Pa s, measured at 20°C, 1013.25 mbar, with a SVM™ 3001 Stabinger Viscometer™ from Anton Paar according to ASTM D7042.

2. The low viscosity oral, dental care and dental cleansing composition according to claim 1, wherein the proportion of the at least one cleaning agent in the composition is 20 to 33% by weight.

3. The low viscosity oral, dental care and dental cleansing composition according to claim 1 or claim 2, wherein the at least one active substance is at least one fluoride active substance, at least one means for gingival care and/or at least one disinfectant component.

4. The low viscosity oral, dental care and dental cleansing composition according to claim 3, wherein the disinfectant component is a bactericide.

5. The low viscosity oral, dental care and dental cleansing composition according to any one of the preceding claims, wherein the proportion of the at least one colour pigment in the composition is 0.3 to 1.0% by weight.

6. The low viscosity oral, dental care and dental cleansing composition according to any one of the preceding claims, wherein the proportion of the at least one flavouring agent in the composition is 0.5 to 1.5% by weight.

7. The low viscosity oral, dental care and dental cleansing composition according to any one of the preceding claims, wherein the solvent is water.

8. Foam obtainable from the composition according to any one of claims 1 to 7.

9. A container comprising
- the composition according to any one of claims 1 to 7,
- a conveying system for conveying the composition outwards,
- a valve, and
- a foam nozzle.

10. The container according to claim 9, wherein the conveying system conveys the composition by means of a propellant gas, compressed air, or via a pumping system.

## Revendications

1. Composition de soin buccal et dentaire et de nettoyage dentaire à basse viscosité, comprenant :
- de 20 à 40 % en poids d'au moins un agent nettoyant,
- de 30 à 45 % en poids d'au moins un agent humectant, l'agent humectant étant choisi dans le groupe
comprenant la glycérine, le sorbitol, le xylitol et de quelconques mélanges de ceux-ci,
- de 0,8 à 3,5 % en poids d'au moins un régulateur de viscosité et d'un agent moussant,
- de 0,5 à 1,8 % en poids d'au moins un stabilisateur de mousse,
- de 3,5 à 8 % en poids d'au moins un principe actif,
- de 0 à 1,0 % en poids d'au moins un pigment coloré,
- de 0 à 1,5 % en poids d'au moins un arôme, et
- des solvants ad 100 % en poids,
la composition faisant preuve d'une viscosité de l'ordre de 50 à 10.000 mPas, mesurée à 20 °C, à 1013,25 mbar, à l'aide d'un viscosimètre SVM™ 3001 Stabinger™ d'Anton Paar selon ASTM D7042.

2. Composition de soin buccal et dentaire et de nettoyage dentaire à basse viscosité selon la revendication 1, la part de l'au moins un agent nettoyant étant de 20 à 33 % en poids de la composition.

3. Composition de soin buccal et dentaire et de nettoyage dentaire à basse viscosité selon la revendication 1 ou la revendication 2, l'au moins un principe actif étant au moins un principe actif fluoré, au moins un agent de soin gingival et/ou au moins un composant désinfectant.

4. Composition de soin buccal et dentaire et de nettoyage dentaire à basse viscosité selon la revendication 3, le composant désinfectant étant un bactéricide.

5. Composition de soin buccal et dentaire et de nettoyage dentaire à basse viscosité selon l'une quelconque des revendications précédentes, la part de l'au moins un pigment coloré étant de 0,3 à 1,0 % en poids de la composition.

6. Composition de soin buccal et dentaire et de nettoyage dentaire à basse viscosité selon l'une quelconque des revendications précédentes, la part de l'au moins un arôme étant de 0,5 à 1,5 % en poids de la composition.

7. Composition de soin buccal et dentaire et de nettoyage dentaire à basse viscosité selon l'une quelconque des revendications précédentes, le solvant étant de l'eau.

8. Mousse, susceptible d'être obtenue à partir de la composition selon l'une quelconque des revendications 1 à 7.

9. Récipient, comprenant
- la composition selon l'une quelconque des revendications 1 à 7,
- un système de convoyage, destiné à convoyer la composition vers l'extérieur,
- une soupape, et
- une buse productrice de mousse.

10. Récipient selon la revendication 9, le système de convoyage convoyant la composition au moyen d'un gaz propulseur, d'air comprimé ou via un système de pompage.
